# EUROPEAN PATENT SPECIFICATION

(11) **EP 1 225 842 B1**
(45) Date of publication and mention of the grant of the patent: **02.05.2003**
(21) Application number: 00977797.0
(22) Date of filing: 31.10.2000
(51) Int. Cl.: A61D 1/02, A61M 5/20

(54) **APPARATUS FOR THE INOCULATION OF MEDICATIONS IN ANIMALS**
GERÄT ZUR MEDIKAMENTENINJEKTION BEI TIEREN
DISPOSITIF SERVANT A INOCULER DES MEDICAMENTS A DES ANIMAUX

(30) Priority: 05.11.1999 IT MI992320
(43) Date of publication of application: 31.07.2002
(73) Proprietor: Lorini, Dante, Crema (IT)
(72) Inventor: Lorini, Dante, Crema (IT)
(74) Representative: Raimondi, Alfredo, Dott. Ing. Prof.
(86) International application number: IB0001774
(87) International publication number: WO01032098

(56) References cited:
- US-A- 3 016 897
- US-A- 3 353 537
- US-A- 4 392 859

## Description

The present invention relates to an apparatus for the inoculation of medication such as vaccines and the like into the muscle of an animal, which comprises a handle supporting devices for pushing the plunger of the syringe, which can be actuated by associated operating means enabled by the operator.

It is known in the technical sector relating to the rearing of animals such as chickens, turkeys and the like that there exists the need to subject the said animals to numerous operations involving the administration of vaccines, antibiotics and medicinal substances in general.

It is also known that these administering operations are performed by means of inoculation into the muscle, for example into the animal's chest, carried out using special syringes which are provided with a handle and the plunger of which is pushed by the action of the operator's thumb against the opposing action of a spring which, once the injection has been performed, causes the return of the plunger into the initial position for inoculation of a new dose of medication drawn from a reservoir as a result of the vacuum effect produced by the said return movement of the plunger.

Although performing their function, these manual syringes have certain drawbacks arising from the fact that the operator is obliged to exert, for each inoculation, a force such as to overcome the resistance of the medication to outward flow from the syringe and penetration into the animal's muscle, as well as the opposing action of the said return spring of the plunger.

Since, during the course of a day, several hundred inoculations are performed, the operator has to make a considerable physical effort which cannot remain constant for the whole of the time necessary for treatment of all the animals, resulting in tiredness, with a consequent increase in the risk of accidents, lack of precision when inserting the needle into the animal's muscle, approximation in the quantity of inoculated medication, which may also be insufficient, and a reduced number of inoculations carried out during the course of the working day.

Such prior art is disclosed into US-A-3 353 537, US-A-4 392 859 and US-A-3 016 897.

The technical problem which is posed, therefore, is that of providing an apparatus for the inoculation of medication such as vaccines and the like in animals, which allows a reduction in the operating force required by the operator, making the quantity of medication inoculated independent of the action of the said operator.

Within the scope of this problem a further requirement is that the apparatus should have a reduced weight and dimensions so that it may be easily handled.

In addition, the apparatus will preferably be provided with safety devices designed to avoid exposure of the needle, outside of actual inoculation, in order to increase the safety for the operator.

These technical problems are solved according to the present invention by an apparatus for the inoculation of medication such as vaccines and the like according to the features of claim 1.

Further details may be obtained from the following description of a non-limiting example of embodiment of the invention provided with reference to the accompanying drawings in which:
- Figure 1 shows a perspective view of an apparatus according to the prior art;
- Figure 2 shows a plan view of the apparatus according to Fig. 1;
- Figure 3 shows a side view of the apparatus according to Fig. 1;
- Figure 4 shows a side view partially sectioned along a longitudinal plane of an embodiment of the apparatus according to the invention.

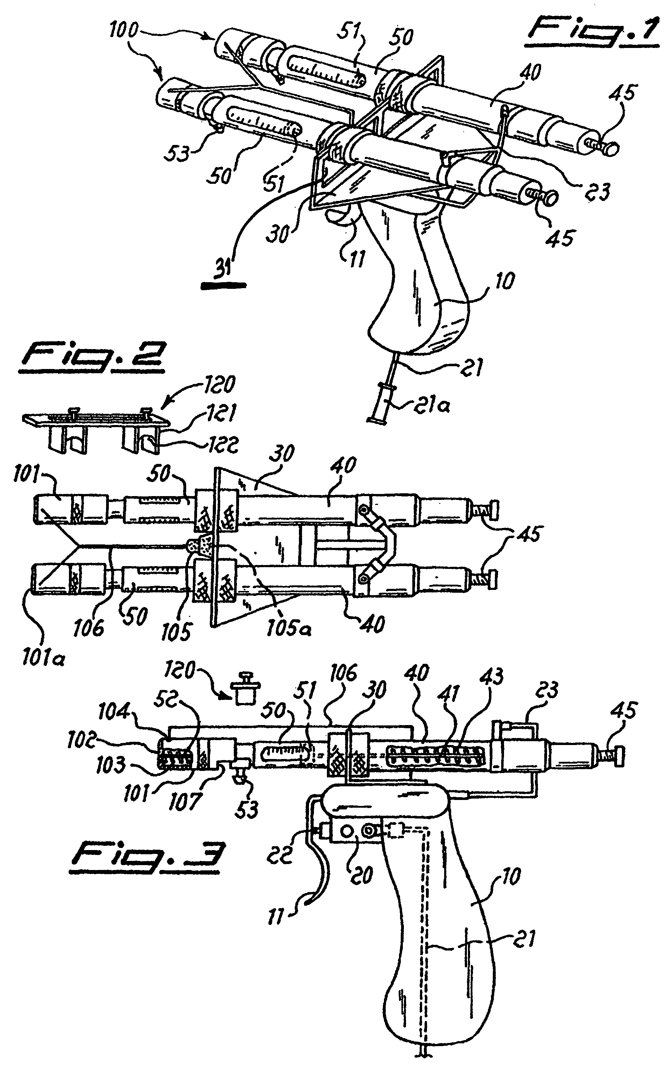

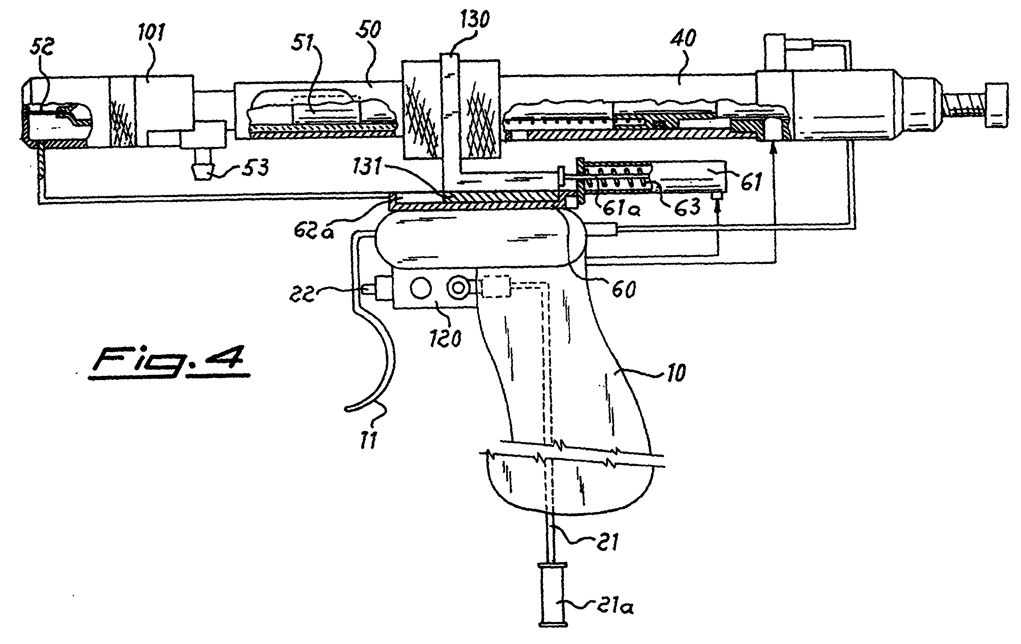

As illustrated (see Figs. 1, 2 and 3), the apparatus according to the prior art is composed essentially of a handle 10, forming the part for gripping by the operator as well as the base for a pneumatic distribution valve 20 and for a fixed support 30 on which there are mounted two pneumatic cylinders 40 for actuating the plunger 51 of a syringe 50 at the free end of which the inoculation needle 52 is arranged.

Below, for the sake of convenience, an orientation of the apparatus will be assumed where the front part is that corresponding to the part with the needle 52 (Fig. 3), while the rear part will be that opposite to the latter.

In greater detail, said handle 10 has an ergonomic shape for firm gripping by the operator and has, passing through it in the axial direction (vertically in the Figure), a pipe 21 supplying the valve 20 with the air under pressure coming from a reservoir (not shown) in turn connected to the said pipe by fast-engagement means 21a.

The air under pressure reaches the distribution valve which, in the embodiment according to Figs. 1-3, is of the two-way type and can be operated by means of a pushbutton 22 which can in turn be actuated by a trigger 11 pivotably mounted on the handle 10 and squeezed by the operator's finger.

The pressure on the pushbutton 22 opens the duct 23 supplying the cylinder 40 which has, arranged inside it, the associated piston 41, the front end of which is integral with the plunger 51 of the syringe 50 inside which the needle 52 is axially inserted.

The syringe 50 also has, radially fitted to it, a nozzle 53 which is in turn connected to a reservoir containing the medication to be inoculated.

A spring 43 is arranged coaxially with the piston 41, the action of said spring causing the return of the said piston into the retracted position.

The rear part of the cylinder 40 is also provided with means 45 for adjusting the stroke of the plunger 51 and therefore the dose of medication to be inoculated.

It is envisaged that the apparatus is also provided with safety means 100 consisting of a sleeve 101 fitted onto the front free end of the syringe 50 with the intervening arrangement of a coaxial spring 103.

The sleeve 101 has a hole 102 in its front surface 101a, said hole, once assembly has been performed, being substantially coaxial with the needle 52 of the syringe; the side surface of the sleeve also has, formed in it, a seat 107 extending in the axial direction from the rear edge thereof and designed to allow coaxial translation without interference of the sleeve relative to the syringe.

It is also envisaged that the sleeve 101 is provided with a second - substantially radial - hole 104 which is formed in the side surface of the said sleeve and into which a pipe 106 supplying a disinfectant 105a drawn from a reservoir 105 may be inserted, as will emerge more clearly below.

Said reservoir, since it has small dimensions, could in turn be integral with the support 30 or the handle 10 or be inside the latter.

The operating principle of the apparatus is as follows: In the rest condition:
- the pushbutton 22 keeps the trigger 11 in the forward position;
- the spring 43 keeps the piston 41 retracted, said piston in turn drawing back the plunger 51 of the syringe 50;
- the spring 103 keeps the sleeve 101 in the forward position so that the needle 52 is covered; during inoculation the operator pushes manually the apparatus against the animal's chest, causing:

- the stopping contact movement of the sleeve 101 and exposure of the needle 52 which is able to penetrate into the animal's muscle with the same thrust exerted by the operator;
- at the same time the operator pulls the trigger 11 which presses the pushbutton 22, which opens the supply of compressed air towards the cylinder 50;
- the pressure of the air, overcoming the opposition of the spring 43, causes the forward movement of the piston 41 and therefore of the plunger 51 which causes entry of the medication into the needle 52 and, from here, into the animal's muscle;
- at the end of inoculation, the valve 20 closes again the air supply, discharging the cylinder 40 and allowing the spring 43 to push the piston 41 and the plunger 51 backwards again;
- the retraction of the plunger 51 produces a vacuum inside the chamber of the syringe 50, which causes the drawing of a new dose of medication for the next inoculation;
- part of the air discharged from the cylinder 40 may also be used in order to place under pressure the reservoir 105 containing the disinfectant 105a which is thus supplied to the needle 52 which is disinfected for the next inoculation.

As illustrated in Fig. 2, auxiliary means 120' for blocking the axial travel of the sleeve 101 are also envisaged so that the latter is unable to retract, exposing the needle during a mistaken movement by the operator who is thus protected against accidental pricks from an infected needle.

Said blocking means may be formed by an auxiliary bridge-piece 121 provided with seats 122 for insertion in the syringe in a position behind the sleeve 101 so. that the latter is no longer able to slide axially backwards.

The blocking means may also consist of an annular rim arranged on the external surface of the sleeve 101 and rotatable on the latter in both directions through a predefined angle so as to bring a suitable projection into a position of interference/non-interference with the nozzle 53 so as to prevent/allow axial sliding of the said sleeve.

In an embodiment of the apparatus according to the invention (Fig. 4) it is envisaged that the support 130 for the assembly consisting of syringe 50/cylinder 40 is provided with longitudinal rails 131 suitable for engagement with respective guides 62a on a platform 60 integral with the handle 10.

In this way, the support 130 is able to be displaced in the longitudinal direction with respect to the platform 60 upon operation of respective actuating means consisting of a pneumatic cylinder 61 connected to the distribution valve 120 which in this case will be of the three-way type and comprising a return spring 63 coaxially arranged around the piston 61a.

In this configuration it is envisaged, moreover, that the sleeve 101 is integral with the platform 60 and locked with the latter during the actuating sequence which is such that the operator need only rest the sleeve 101 against the animal's muscle and press the trigger 11 which, actuating the pushbutton 22, causes initially the supply of air under pressure to the cylinder 61 and therefore advancing of the support 130 with the syringe/cylinder assembly with respect to the platform 60, so as to move the needle 52 outside of the sleeve 101 and into the animal's muscle; at this point the pushbutton 22 activates the above-described cycle which inoculates the medication by means of the syringe needle.

It is therefore evident how, with this second embodiment, the operator does not have to exert any force even to insert the needle into the animal's muscle, which insertion, being performed by automatic means, may be conveniently calibrated at the start of the working period depending on the kind and/or size of animal to be treated and kept constant and repeatable until the last animal in the batch undergoing treatment.

It is therefore evident how the apparatus according to the invention allows inoculation to be performed without substantial effort by the operator who, operating the trigger, merely has to overcome the resistance of the pushbutton 22 which may be set to very low values, thus transferring all the actuating force of the syringe plunger to the air pressure, said pressure being in turn determined and calibrated by corresponding external means such as a compressor, a pressure reservoir or the like, with associated means for controlling and measuring the correct calibration.

This allows inoculation to be performed in a substantially automatic manner without fatigue on the part of the operator, resulting in reliable metering of the inoculated dose which no longer depends on the skill and on the physical condition of the operator.

In addition to this and in view of the particular operating conditions, the general safety of the operation is also greatly improved since the operator must merely perform the action of pressing the needle into the muscle of the animal, resulting in a reduction in the number of movements to be performed. Consequently the productivity of the operation is also greatly improved, allowing a much higher number of inoculations compared to the previous devices. As can be understood, the above description has been provided in connection with only one of the two syringes illustrated, it being obvious that the minimum configuration of the apparatus envisages only one syringe. As illustrated, however, it is possible to arrange several syringes in parallel for the simultaneous -inoculation of different medication or for doubling the dose of a single medication. It is envisaged that each syringe and the associated cylinder are arranged inside a respective eyelet 31 of the support 30,130, which allow the two syringes to be positioned along interaxes suitable for the specific size of the animal.

## Claims

1. Apparatus for the inoculation of medication such as vaccines and the like into the muscle of an animal, which comprise a handle (10) supporting at least one syringe (50) with associated needle (52), devices (40) for pushing the plunger (51) of the syringe (50), which can be actuated by associated operating means (120) enabled by the operator, **characterized in that** it also comprises means (60,62a) fixed to the handle (10) and suitable to cooperate with corresponding means (130,131) of the syringe/cylinder assembly for the longitudinal displacement of the latter, said operating means (120) being able to determine in sequence both the displacement of the syringe/cylinder assembly in respect of the handle (10) and the displacement of the plunger (51) in respect of the syringe (50).

2. Apparatus according to Claim 1, **characterized in that** said means integral with the handle (10) are a platform (60) provided with longitudinal rails (62a) suitable for engagement with corresponding guides (131) of a support (130) of the syringe/cylinder assembly.

3. Apparatus according to Claim 2, **characterized in that** said support (130) is movable translationwise in the longitudinal direction with respect to the platform (60).

4. Apparatus according to Claim 3, **characterized in that** it comprises respective means for actuation in the longitudinal direction of the support (130).

5. Apparatus according to Claim 4, **characterized in that** said actuating means consist of a pneumatic cylinder (61) connected to the distribution valve (120) and comprising a return spring (63) coaxially arranged around the piston (61a).

6. Apparatus according to Claim 1, **characterized in that** said pushing devices consist of a pneumatic cylinder (40), the piston (41) of which is coaxially connected to the plunger (51) of the syringe (50).

7. Apparatus according to Claim 6, **characterized in that** resilient opposition means (43) are arranged coaxially with said piston (41) and are designed to keep the piston (41) in the retracted position.

8. Apparatus according to Claim 6, **characterized in that** said operating means consist of a valve (20) for distributing a fluid under pressure for actuating the piston (41) of the cylinder (40) against the opposition action of said resilient means.

9. Apparatus according to Claim 1, **characterized in that** said operating means comprise an activation pushbutton (22) arranged in contact with a trigger (12) pivotably mounted on the handle (10).

10. Apparatus according to Claim 1, **characterized in that** said cylinder (40) comprises coaxial means (45) for adjusting the stroke of the plunger (51) of the syringe (50).

11. Apparatus according to Claim 1, **characterized in that** it envisages safety means (100) for containing the needle (52).

12. Apparatus according to Claim 11, **characterized in that** said safety means (100) consist of a sleeve (101) coaxially mounted on the front free end of the syringe (50).

13. Apparatus according to Claim 12, **characterized in that** said sleeve (101) comprises a first hole (101a) formed on its front surface in a position coaxial with the needle (52) so as to allow the latter to protrude.

14. Apparatus according to Claim 12, **characterized in that** said sleeve (101) has at least one radial hole (104) formed on its side surface.

15. Apparatus according to Claim 12, **characterized in that** the sleeve (101) is integral with the platform (60) and locked therewith.

16. Apparatus according to Claim 12, **characterized in that** a seat (107) is present on the side surface of said sleeve (101), said seat extending in an axial direction from the rear edge thereof and being designed to allow coaxial translation of the syringe with respect to the sleeve.

17. Apparatus according to Claim 1, **characterized in that** it comprises means for disinfecting the needle (52).

18. Apparatus according to Claim 17, **characterized in that** said disinfecting means consist of a pipe (106) inserted into said radial hole (104) of the sleeve (101) and connected to a reservoir (105) for the disinfectant (105a).

19. Apparatus according to Claim 17, **characterized in that** said disinfectant reservoir (105) comprises means for connection to the distribution valve (20), designed to convey the fluid under pressure inside the said reservoir.

20. Apparatus according to Claim 19, **characterized in that** said fluid for pressurisation of the disinfectant reservoir consists partly of the fluid recovered during the return movement of the piston (41) into the rest position.

21. Apparatus according to the preceding claims, **characterized in that** it comprises a plurality of syringes (50).

22. Apparatus according to Claim 21, **characterized in that** said syringes are housed in transversely elongate seats (31) of the support (30;130) integral with the handle (10), designed to allow adjustment of the interaxial distance between the needles (52).

## Patentansprüche

1. Vorrichtung zum Einimpfen von Medikamenten wie Impfstoffen und dergleichen in den Muskel eines Tieres, die einen Griff (10) aufweist, der mindestens eine Spritze (50) mit zugehöriger Nadel (52) trägt, Einrichtungen (40) zum Stoßen des Kolbens (51) der Spritze (50), die mittels zugeordneter Betätigungsmittel (120) durch die Bedienungsperson aktiviert werden können, **dadurch gekennzeichnet, dass** sie außerdem am Griff (10) befestigte Mittel(60,62a) aufweist, die mit entsprechenden Mitteln (130, 131) der Spritze/Zylinder-Anordnung für die Längsverschiebung der letzteren zusammenarbeiten kann, wobei die Betätigungsmittel (120) aufeinanderfolgend sowohl die Verschiebung der Spritze/Zylinder-Anordnung bezüglich des Griffs (10) als auch die Verschiebung des Kolbens (51) bezüglich der Spritze bestimmen können.

2. Vorrichtung nach Anspruch 1, **dadurch gekennzeichnet, dass** die mit dem Griff (10) integralen Mittel eine Plattform (60) sind, die mit Längsschienen (62a) versehen ist, die zum Eingriff mit entsprechenden Führungen (131) eines Trägers (130) der Spritze/ Zylinder-Anordnung geeignet sind.

3. Vorrichtung nach Anspruch 2, **dadurch gekennzeichnet, dass** der Träger (130) zur Verschiebung in Längsrichtung bezüglich der Plattform (60) beweglich ist.

4. Vorrichtung nach Anspruch 3, **dadurch gekennzeichnet, dass** sie entsprechende Mittel zur Betätigung in der Längsrichtung des Trägers (130) umfasst.

5. Vorrichtung nach Anspruch 4, **dadurch gekennzeichnet, dass** die Mittel zur Betätigung aus einem pneumatischen Zylinder (61) bestehen, der mit dem Verteilerventil (120) verbunden ist und eine Rückstellfeder (63) umfasst, die koaxial um den Kolben (61a) angeordnet ist.

6. Vorrichtung nach Anspruch 1, **dadurch gekennzeichnet, dass** die Stoßvorrichtungen aus einem pneumatischen Zylinder (40) bestehen, dessen Kolben (41) koaxial mit dem Kolben (51) der Spritze (50) verbunden ist.

7. Vorrichtung nach Anspruch 6, **dadurch gekennzeichnet, dass** elastische Widerstandsmittel (43) koaxial um den Kolben (41) angeordnet und so ausgestaltet sind, dass sie den Kolben (41) in der zurückgezogenen Position halten.

8. Vorrichtung nach Anspruch 6, **dadurch gekennzeichnet, dass** die Betätigungsmittel aus einem Ventil (20) bestehen, das eine Flüssigkeit unter Druck verteilt, um den Kolben (41) des Zylinders (40) gegen den Widerstand der elastischen Mittel zu betätigen.

9. Vorrichtung nach Anspruch 1, **dadurch gekennzeichnet, dass** die Betätigungsmittel einen Aktivierungs-Druckknopf (22) umfassen, der in Kontakt mit einem Auslöser (12) angeordnet ist, welcher schwenkbar an dem Griff(10) angebracht ist.

10. Vorrichtung nach Anspruch 1, **dadurch gekennzeichnet, dass** der Zylinder (40) koaxiale Mittel (45) umfasst, die den Hub des Kolbens (51) der Spritze (50) regulieren.

11. Vorrichtung nach Anspruch 1, **dadurch gekennzeichnet, dass** sie Sicherheitsmittel (100) zur Aufnahme der Nadel (52) umfasst.

12. Vorrichtung nach Anspruch 11, **dadurch gekennzeichnet, dass** die Sicherheitsmittel (100) aus einer Hülse (101) bestehen, die koaxial an dem vorderen freien Ende der Spritze (50) angeordnet sind.

13. Vorrichtung nach Anspruch 12, **dadurch gekennzeichnet, dass** die Hülse (101) an ihrer Frontfläche ein erstes Loch (101a) umfasst, das koaxial zu der Nadel (52) gebildet ist, sodass letztere aus ihm heraustreten kann.

14. Vorrichtung nach Anspruch 12, **dadurch gekennzeichnet, dass** die Hülse (101) mindestens ein auf ihrer Seitenfläche gebildetes radiales Loch (104) aufweist.

15. Vorrichtung nach Anspruch 12, **dadurch gekennzeichnet, dass** die Hülse (101) mit der Plattform (60) eine Einheit bildet und somit verriegelt ist.

16. Vorrichtung nach Anspruch 12, **dadurch gekennzeichnet, dass** eine Aufnahme (107) auf der Seitenfläche der Hülse (101) vorhanden ist, wobei die Aufnahme sich in einer axialen Richtung von ihrer hinteren Kante erstreckt und so ausgebildet ist, dass eine koaxiale Verschiebung der Spritze bezüglich der Hülse möglich ist.

17. Vorrichtung nach Anspruch 1, **dadurch gekennzeichnet, dass** sie Mittel zum Desinfizieren der Nadel (52) umfasst.

18. Vorrichtung nach Anspruch 17, **dadurch gekennzeichnet, dass** die Desinfektionsvorrichtung aus einer Röhre (106) besteht, die in das radiale Loch (104) der Hülse (101) eingeführt und mit einem Behälter (105) für das Desinfektionsmittel (105a) verbunden ist.

19. Vorrichtung nach Anspruch 17, **dadurch gekennzeichnet, dass** der Desinfektionsmittel-Behälter (105) Mittel zur Verbindung mit dem Verteilerventil (20) umfasst, die so ausgebildet sind, dass sie die Flüssigkeit unter Druck in den Behälter einbringen können.

20. Vorrichtung nach Anspruch 19, **dadurch gekennzeichnet, dass** das Fluid, das Druck auf den Desinfektionsmittel-Behälter ausübt, teilweise aus dem Fluid besteht, das während der Zurückbewegung des Kolbens (41) in die Ruheposition gewonnen wird.

21. Vorrichtung nach einem der vorgehenden Ansprüche, **dadurch gekennzeichnet, dass** sie mehrere Spritzen (50) enthält.

22. Vorrichtung nach Anspruch 21, dass die Spritzen in sich quer erstreckenden Aufnahmen (31) des Trägers (30; 130) integral mit dem Griff (10) untergebracht und so ausgebildet sind, dass eine Einstellung des Abstands zwischen den Achsen der Nadeln (52) möglich ist.

## Revendications

1. Appareil pour l'inoculation de médications tels des vaccins et produits similaires dans le muscle d'un animal, qui comprend une poignée (10) supportant au moins une seringue (50) et son aiguille (52), des dispositifs (40) permettant de pousser le piston (51) de la seringue (50), qui peut être actionné par divers moyens de fonctionnement (120) mis en oeuvre par l'opérateur, **caractérisé en ce qu'**il comprend également des moyens (60, 62a) fixés à la poignée (10) et pouvant être utilisés en association avec les moyens correspondants (130, 131) de l'ensemble seringue/cylindre pour le déplacement longitudinal de ce dernier, lesdits moyens de fonctionnement (120) permettant de déterminer en séquence à la fois le déplacement de l'ensemble seringue/cylindre par rapport à la poignée (10) et le déplacement du piston (51) par rapport à la seringue (50).

2. Appareil selon la revendication 1, **caractérisé en ce que** lesdits moyens intégrés à la poignée (10) sont un plateau (60) équipé de rails longitudinaux (62a) permettant la mise en prise avec des guides correspondants (131) d'un support (130) de l'ensemble seringue/cylindre.

3. Appareil selon la revendication 2, **caractérisé en ce que** ledit support (130) peut subir un mouvement de translation dans le sens longitudinal par rapport au plateau (60).

4. Appareil selon la revendication 3, **caractérisé en ce qu'**il comprend des moyens respectifs permettant de l'actionner dans le sens longitudinal du support (130).

5. Appareil selon la revendication 4, **caractérisé en ce que** lesdits moyens d'actionnement consistent en un cylindre pneumatique (61) relié à la valve de distribution (120) et comprenant un ressort de rappel (63) entourant coaxialement le piston (61a).

6. Appareil selon la revendication 1, **caractérisé en ce que** lesdits poussoirs comprennent un cylindre pneumatique (40), dont le piston (41) est relié coaxialement au piston (51) de la seringue (50).

7. Appareil selon la revendication 6, **caractérisé en ce que** des moyens d'opposition résilients (43) entourent coaxialement ledit piston (41) et sont conçus pour que le piston (41) soit maintenu dans sa position rétractée.

8. Appareil selon la revendication 6, **caractérisé en ce que** lesdits moyens de fonctionnement comprennent une valve (20) pour la distribution d'un fluide sous pression permettant d'actionner le piston (41) du cylindre (40) contre la force d'opposition appliquée desdits moyens de résilience.

9. Appareil selon la revendication 1, **caractérisé en ce que** lesdits moyens de fonctionnement comprennent un bouton-poussoir d'activation (22) disposé en contact avec une détente (12) montée sur la poignée (10) de façon à pouvoir pivoter.

10. Appareil selon la revendication 1, **caractérisé en ce que** ledit cylindre (40) comprend des moyens coaxiaux (45) permettant d'ajuster l'actionnement du piston (51) de la seringue (50).

11. Appareil selon la revendication 1, **caractérisé en ce qu'**il comporte des moyens de sécurité (100) pour le stockage de l'aiguille (52).

12. Appareil selon la revendication 11, **caractérisé en ce que** lesdits moyens de sécurité (100) comprennent un manchon (101) monté coaxialement sur l'extrémité libre avant de la seringue (50).

13. Appareil selon la revendication 12, **caractérisé en ce que** ledit manchon (101) comprend un premier trou (101a) sur sa face avant dans une position coaxiale par rapport à l'aiguille (52) afin de permettre à cette dernière de sortir.

14. Appareil selon la revendication 12, **caractérisé en ce que** ledit manchon (101) comporte au moins un trou radial (104) formé sur sa face latérale.

15. Appareil selon la revendication 12, **caractérisé en ce que** ledit manchon (101) forme un seul tenant avec le plateau (60) et est verrouillé à celui-ci.

16. Appareil selon la revendication 12, **caractérisé en ce qu'**une butée (107) est présente sur la face latérale dudit manchon (101), ladite butée sortant dans une direction axiale depuis l'arête arrière de celui-ci et étant conçue pour permettre la translation coaxiale de la seringue par rapport au manchon.

17. Appareil selon la revendication 1, **caractérisé en ce qu'**il comprend des moyens de désinfection de l'aiguille (52).

18. Appareil selon la revendication 17, **caractérisé en ce que** lesdits moyens de désinfection comprennent un tuyau (106) inséré dans ledit trou radial (104) du manchon (101) et relié à un réservoir (105) contenant le désinfectant (105a).

19. Appareil selon la revendication 17, **caractérisé en ce que** ledit réservoir de désinfectant (105) comprend des moyens de connexion à la valve de distribution (20), conçus pour acheminer le fluide sous pression dans ledit réservoir.

20. Appareil selon la revendication 19, **caractérisé en ce que** ledit fluide pour la pressurisation du réservoir de désinfectant est constitué en partie du fluide récupéré pendant le mouvement de retour du piston (4b) dans sa position de repos.

21. Appareil selon les revendications précédentes, **caractérisé en ce qu'**il comprend plusieurs seringues (50).

22. Appareil selon la revendication 21, **caractérisé en ce que** lesdites seringues sont stockées dans des butées (31) allongées transversalement du support (30, 130), formant un seul tenant avec la poignée (10), conçues pour permettre le réglage de la distance interaxiale entre les aiguilles (52).
